# EUROPEAN PATENT SPECIFICATION

(11) **EP 3 634 415 B1**
(45) Date of publication and mention of the grant of the patent: **11.02.2026**
(21) Application number: 18813969.5
(22) Date of filing: 07.06.2018
(51) Int. Cl.: A61K 31/498, A61P 27/06, A61K 47/02, A61K 9/08, A61K 31/407, A61K 31/4535, A61K 31/5355, A61K 31/5575, A61K 47/12, A61K 47/26, A61K 47/38, A61K 9/00, A61P 37/08

(54) **OPHTHALMOLOGICAL COMPOSITIONS COMPRISING BRIMONIDINE AND KETOTIFEN FOR USE IN THE TREATMENT OF ALLERGIES**
OPHTALMOLOGISCHE ZUBEREITUNGEN ENTHALTEND BRIMONIDIN UND KETOTIFEN ZUR VERWENDUNG BEI DER BEHANDLUNG VON ALLERGIEN
COMPOSITIONS OPHTALMOLOGIQUES COMPRENANT DE LA BRIMONIDINE ET DU KÉTOTIFÈNE POUR UNE UTILISATION DANS LE TRAITEMENT DES ALLERGIES

(30) Priority: 08.06.2017 US 201762516931 P; 24.01.2018 US 201862621082 P
(43) Date of publication of application: 15.04.2020
(60) Divisional application: 25225699.5
(73) Proprietor: Eye Therapies, LLC, Dana Point, CA 92654 (US)
(72) Inventor: HORN, Gerald, Deerfield IL 60015 (US)
(74) Representative: Elkington and Fife LLP
(86) International application number: PCT/US2018/036440
(87) International publication number: WO 2018/226942

(56) References cited:
- WO-A1-2015/138786
- WO-A2-2008/136034
- US-A1- 2010 028 266
- US-A1- 2010 240 625
- US-A1- 2016 193 194
- US-A1- 2016 303 119
- US-A1- 2016 354 383
- ONO, SJ ET AL.: "Allergic conjunctivitis: Update on pathophysiology and prospects for future treatment", JOURNAL OF ALLERGY AND CLINICAL IMMUNOLOGY, vol. 115, no. 1, January 2005 (2005-01-01), pages 118 - 122, XP004701626
- RASCHE, S ET AL.: "Tmem16b is Specifically Expressed in the Cilia of Olfactory Sensory Neurons", CHEMICAL SENSES, vol. 35, no. 3, 25 January 2010 (2010-01-25), pages 239 - 245, XP055555675

## Description

### FIELD OF THE INVENTION

The present invention is related to compositions containing low-dose brimonidine and an anti-histamine. The present invention is further related to methods of treating allergies by administering compositions of the present invention. Any references in the description to methods of treatment refer to the compounds, pharmaceutical compositions and medicaments of the invention for use in a method of treatment of the human body by therapy.

### BACKGROUND OF THE INVENTION

Allergic diseases are a major health problem worldwide. Furthermore, the prevalence of allergic disease is increasing in most countries. Two major forms of allergies include allergic rhinitis and ocular allergies. Allergic reactions occur, in part, when the immune system reacts to the presence of an allergen and produces histamine. Histamine then causes the major symptoms of allergies including a "runny" or "itchy" nose, sneezing, "watery" eyes, itchy throat, etc.

In allergic rhinitis, the allergic reaction occurs in the nose. Histamine released in response to the reaction causes local vascular dilation, with resultant increased capillary pressure as well as increased capillary permeability. Both of these effects cause rapid fluid leakage into the tissues of the nose and the nasal linings become swollen and secretory.

Ocular allergies or allergic conjunctivitis often accompanies allergic rhinitis in response to the release of histamines. Ocular signs and symptoms of allergies include itching, redness, swelling, tearing, burning and stinging.

Ocular allergies are often treated with eye drops including Opcon-A^{®} (0.02675% naphazoline hydrochloride and 0.315% pheniramine maleate; Opcon A is a registered trademark of Bausch & Lomb Incorporated), Naphcon-A^{®} (0.025% naphazoline hydrochloride and 0.3% pheniramine maleate; Naphcon-A is a registered trademark of Alcon Research Ltd), and Vasocon^{®} (0.05% naphazoline hydrochloride and 0.5% antazoline phosphate; Vasocon is a registered trademark of Novartis AG) and Albalon^{™} (0.05% naphazoline hydrochloride, Albalon is available from Allergan Pharmaceuticals).

However, many of the anti-histamines prescribed or administered to treat ocular allergies and or allergic rhinitis treat histamine induced itching and discomfort but do not fully alleviate the attendant symptoms including particularly swelling secondary to vascular leakage and hyperemia (eye redness) resulting in both remaining discomfort and an unhealthy appearance. Thus, there is a further need in the art for a combination allergy treatment that leads to a greater alleviation of attendant symptoms including eye redness.
US 2010/028266 relates to compositions and methods for treating and/or preventing an allergic response with reduced rebound hyperemia, comprising highly selective alpha-2 adrenergic receptor agonists, at low concentrations, such as below 0.05% weight by volume, and histamine antagonists. The compositions preferably comprise brimonidine. The compositions preferably have pH between about 5.5 and about 6.5.
US 2010/240625 relates to stable topical formulations of cetirizine that provide a comfortable formulation when instilled in the eye and is effective in the treatment of allergic conjunctivitis and/or allergic conjunctivitis. US 2010/240625 further relates to methods of treating allergic conjunctivitis and/or allergic rhinoconjunctivitis in a subject in need of such treatment by topical application of the cetirizine formulations directly to the eye.

### SUMMARY OF THE INVENTION

The present invention is directed to an ophthalmological composition for the treatment of allergies comprising brimonidine at a concentration from 0.025% to 0.035% w/v and ketotifen at a concentration from 0.025% to 0.7% w/v, wherein w/v denotes weight by total volume of the composition. Ketotifen is a histamine antagonist.

In another embodiment, the present invention is directed to a composition of the present invention for use in a method of treating or preventing allergies comprising administering the composition of the present invention to a subject in need thereof.

### BRIEF DESCRIPTION OF THE DRAWINGS

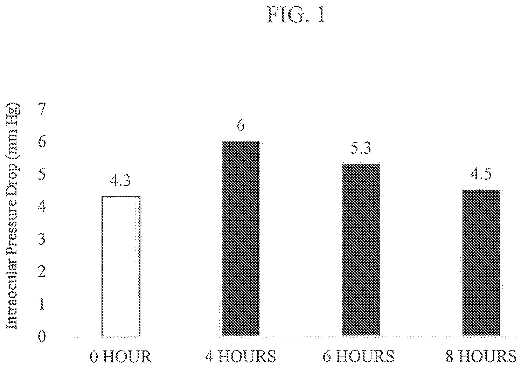

Figure 1- a prophetic example of IOP reduction after administration of 0.005% latanoprost (0 hours, white bar), versus IOP reduction after administration of 0.03% brimonidine and 0.005% latanoprost (4, 6 and 8 hours, black bar).

### DETAILED DESCRIPTION OF THE INVENTION

It is a discovery of the present invention that postcapillary venular leakage may be reduced selectively by low dose brimonidine over alpha 1 vasoconstrictors. Not wishing to be held to particular theory, it is believed alpha 1 agonists constrict larger vessels such as arterioles to a much greater extent than low dose brimonidine. The constriction of the larger vessels creates ischemia, and with repeated use, inflammation and rebound hyperemia. The leakage in postcapillary venules has been shown to be related to transient loss of vascular endothelial VE cadherin protein resulting in gap junctions with leakage along the postcapillary venular cell wall. However, constriction of small vessels and particularly said postcapillary venules without ischemia by low dose brimonidine is surprisingly found to reduce such leakage without the ischemia of tetrahydrozaline, oxymetazoline, or other alpha 1 agonist constrictors, alleviating redness and clinical sequelae of swelling and redness otherwise not as completely alleviated making the combination of low dose brimonidine with an anti-histamine particularly effective.

The term "brimonidine" encompasses, without limitation, brimonidine and brimonidine salts, and specifically includes, but is not limited to, brimonidine tartrate, 5-bromo-6-(2-imidazolin-2-ylamino)quinoxaline D-tartrate, Alphagan^{®} (Alphagan is a registered trademark of Allergan, Inc.), and UK14,304.

The term "glaucoma drug" or "second glaucoma drug" encompasses, without limitation, all drugs used to treat glaucoma including those drugs used to lower intraocular pressure with the exception of brimonidine.

The term "histamine antagonist" includes all chemicals that have been found to antagonize at least one histamine receptor including histamine H₁ and histamine H₂.

The term "allergy" or "allergies" or "allergic response" refers to allergic rhinitis and or ocular allergies. Allergic rhinitis is an allergic inflammation of the nasal airways with attendant symptoms, including, but not limited to, rhinorrhea, nasal obstruction, nasal itching, sneezing, ocular pruritis. Ocular allergies are any allergic diseases of the eye, including, but not limited to, seasonal/perennial allergic conjunctivitis, vernal keratoconjunctivitis, giant papillary conjunctivitis, perennial allergic conjunctivitis and atopic keratoconjunctivitis.

The term "pharmaceutically acceptable salts" is meant to include salts of the active compounds which are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either net or in a suitable inert solvent. Examples of pharmaceutically acceptable base addition salts include sodium, potassium, calcium, ammonium, organic amino, or magnesium salt, or a similar salt. When compounds of the present invention contain relatively basic functionalities, acid addition salts can be obtained by contacting the neutral form of such compounds with a sufficient amount of the desired acid, either net or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isbutyric, oxalic, maleic, malonic, benzoic, succinic, suberic, fumeric mandelic, phthalic, benzenesulfonic, p-tolylsulfonic, citric, tartaric, methanesulfonic, and the like. Also included are salts of amino acids such as arginate and the like, and salts of organic acids like glucuronic or galactunoric acids and the like (see, for example, Berge, S. M., et al., "Pharmaceutical Salts", Journal of Pharmaceutical Science, 1977, 66, 1-19). Certain specific compounds of the present inventions contain both basic and acidic functionalities that allow the compounds to be converted into either base or acid addition salts.

The neutral forms of the compounds may be registered by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present invention.

In additional to salt forms, the present invention provides compounds which are in a prodrug form. Prodrugs of the compounds described herein are those compounds that readily undergo chemical changes under physiological conditions to provide the compounds of the present invention. Additionally, prodrugs can be converted to the compounds of the present invention by chemical or biochemical methods in an ex vivo environment. For example, prodrugs can be slowly converted to the compounds of the present invention when placed in a transdermal patch reservoir with a suitable enzyme or chemical reagent. Prodrugs are often useful because, in some situations, they may be easier to administer than the parent drug. They may, be bioavailable by oral administration whereas the parent drug is not. The prodrug may also have improved solubility in pharmacological compositions over the parent drug. A wide variety of prodrug derivatives are known in the art, such as those that rely on hydrolytic cleavage or oxidative activation of the prodrug. An example, without limitation, of a prodrug would be a compound of the present invention which is administered as an ester (the "prodrug"), but then is metabolically hydrolyzed to the carboxylic acid, the active entity. Additional examples include peptidyl derivatives of a compound of the invention.

Certain compounds of the present invention can exist in unsolvated forms as well as solvated forms, including hydrated forms. In general, the solvated forms are equivalent to unsolvated forms and are intended to be encompassed within the scope of the present invention. Certain compounds of the present invention may exist in multiple crystalline or amorphous forms. In general, all physical forms are equivalent for the uses contemplated by the present invention and are intended to be within the scope of the present invention.

In preferred embodiments, compositions of the present invention may comprise a nonionic surfactant, a viscosity enhancer, preferably hydroxypropylmethyl cellulose ("HPMC") or carboxymethyl cellulose, a buffer and optionally, sorbate.

Nonionic surfactants suitable for use in the present invention include, but are not limited to a polysorbate, a poloxamer, a polyoxyl, an alkyl aryl poly ether, a cyclodextrin, a tocopheryl polyethylene glycol succinate. a glucosyl dialkyl ethers and a crown ether, ester-linked surfactants. Nonionic surfactants may be present in compositions of the present invention at a concentration from about 1% to about 5% w/v.

Polysorbates suitable for use in the present invention include, but are not limited to, polysorbate 20 (polyoxyethylene (20) sorbitan monolaurate), polysorbate 40 (polyoxyethylene (20) sorbitan monopalmitate), polysorbate 60 (polyoxyethylene (20) sorbitan monostearate) and polysorbate 80 (polyoxyethylene (20) sorbitan monooleate.

Polysorbates may be present in compositions of the present invention at a concentration from about 1% to about 5% w/v, more preferably from about 2% to about 3% w/v and most preferably at about 2.5% w/v.

Cyclodextrins suitable for use in the present invention include, but are not limited to, ionically charged (e.g. anionic) beta - cyclodextrins with or without a butyrated salt (Captisol^{®}) 2-hydroxypropyl beta cyclodextrin ("HPβCD"), alpha cyclodextrins and gamma cyclodextrins.

Poloxamers include but are not limited to poloxamer 103, poloxamer 123, and poloxamer 124, poloxamer 407, poloxamer 188, poloxamer 338 and any poloxamer analogue or derivative

Polyoxyls include but are not limited to Brij^{®} 35, 78, 98, 700 (polyoxyethylene glycol alkyl ethers) and Spans (sorbitan esters) and Span^{®} 20-80 (sorbitan monolaurate, sorbitan monopalmitate, sorbitan monostearate, and sorbitan monooleate).

The viscosity enhancer, preferably HPMC, may be present in compositions of the present invention at a concentration from about 0.1% to about 1.2% w/v, preferably at about 1.2% w/v.

Buffers useful in the present invention include, but are not limited to, citrate buffer and phosphate buffer. Buffers may be present in compositions of the present invention at a concentration from about 1 millimolar ("mM") to about 10 mM, preferably from about 2 mM to about 6 mM and more preferably from about 3 mM to about 4mM.

Sorbate may be present in compositions of the present invention at a concentration from about 0.01% to about 0.5% w/v, preferably from about 0.05% to about 0.25% w/v and more preferably at about 0.1% w/v.

As used herein, all numerical values relating to amounts, weights, and the like, that are defined as "about" each particular value is plus or minus 10%. For example, the phrase "about 5% w/v" is to be understood as "4.5% to 5.5% w/v." Therefore, amounts within 10% of the claimed value are encompassed by the scope of the claims.

As used herein "% w/v" refers to the percent weight of the total composition.

As used herein the terms "subject" and "patient" are used interchangeably and refer, but are not limited to, a person or other animals.

As used herein, the term "prevent" or "preventing" refers to precluding, averting, obviating, forestalling, stopping, or hindering something from happening, especially by advance action.

As used herein, the term "treat" or "treating" refers to reversing, alleviating or slowing the progress of the disease, disorder, or condition to which such terms apply, or one or more symptoms of such disease, disorder, or condition.

As used herein, the term "administration" or "administering" refers to topical application, injection or administration via implants.

The articles "a," "an" and "the" are intended to include the plural as well as the singular, unless the context clearly indicates otherwise.

### Combination with Histamine Antagonists

Currently available anti-histamines (i.e. histamine antagonists) are not capable of fully alleviating all allergy symptoms including particularly the eye redness associated with cytokine induced vasodilation. It is a discovery of the present invention that the use of extremely low dose brimonidine ("ELDB") in combination with an anti-histamine leads to not only greater reduction of redness but greater alleviation of allergy symptoms such as conjunctival swelling than the use of the anti-histamine alone.

The present invention is directed to an ophthalmological composition for the treatment of allergies comprising brimonidine at a concentration from 0.025% to about 0.035%w/v and ketotifen at a concentration from 0.025% to 0.7% w/v, wherein w/v denotes weight by total volume of the composition. Ketotifen is a histamine antagonist.

Brimonidine may be at concentrations about 0.025%, about 0.035% or about 0.04% w/v.

Histamine antagonists for nasal or ophthalmological administration are currently marketed under the following concentrations of active ingredients: 0.05% naphazoline HCl; combination of 0.05% naphazoline HCl and 0.5% antazoline phosphate; combination of 0.05% naphazoline HCl and 0.3% pheniramine maleate; combination of 0.02675% naphazoline hydrochloride and 0.315% pheniramine maleate; 0.1% and 0.15% azelastine; 0.05% emedastine; 0.05% levocabastine HCl; 0.025% ketotifen; and 0.1%, 0.2% and 0.7% olopatadine.

### EXAMPLES

### Reference Example 1-Prophetic Intraocular Pressure Reduction

### Method

A subject with normo-tensive baseline intraocular pressure ("IOP"; < 21 mm Hg) was prophetically treated for 5 consecutive days with a single instillation of two drops per eye per day of a composition containing 0.035% brimonidine, 0.005% latanoprost, and a combination of 0.03% brimonidine and 0.005% latanoprost. A 1-week washout period was observed between each of the three treatment cycles. Instillation was performed at 8:30 AM, followed by 30 seconds of punctual occlusion with instillation on days 1, 3, and 5.

IOP was measured at one or more of 4 hrs, 8 hrs, 12 hrs, 24 hrs, 32 hrs and comfort and side effect profile were qualitatively assessed.

### Results

Based on preliminary data, the tested formulations prophetically achieved greater IOP reduction than latanoprost alone. Further, a peak IOP reduction effect is prophetically achieved at about 4 to 8 hours after instillation and the IOP remained below the baseline 24 hours after instillation. Figure 1 demonstrates a prophetic example of IOP reduction after administration of 0.005% latanoprost (0 hours, white bar), versus IOP reduction after administration of 0.03% brimonidine and 0.005% latanoprost (4, 6 and 8 hours, black bar). In fact, the reduction in IOP after administration of the combination of ELDB and latanoprost prophetically achieved synergy at all times and days except prior to instillation. Synergy will be calculated by taking the observed reduction in IOP and dividing by the expected reduction in IOP to give a "synergy factor". Synergy factors greater than 1 demonstrate synergy. Expected IOP reduction will be calculated using the following formula where A is the IOP reduction for ELDB alone and B is the IOP reduction for latanoprost alone: A + B - (AB/100). Thus, the formulations of the invention prophetically demonstrate improved performance over full strength brimonidine and latanoprost alone under similar conditions of testing. Finally, during testing of latanoprost alone subject prophetically experienced ocular hyperemia that was not observed during the testing of brimonidine or the brimonidine and latanoprost combination demonstrating that extremely low dose brimonidine is capable of not only enhancing IOP reduction but also reducing attendant side effects of glaucoma drugs.

In conclusion, the combination of ELDB and a second glaucoma drug prophetically provide enhanced and synergistic relief of intraocular pressure that is either as good or better than that provided by the second glaucoma drug alone or the second glaucoma drug in combination with full strength brimonidine. Further, the combination of ELDB and the second glaucoma drug prophetically provide reduced or ameliorated side effects that normally occur when the second glaucoma drug is administered alone.

### Example 2-Prophetic Allergy Relief

### Method

A subject suffering from nasal allergies was prophetically administered a combination of 0.005% to 0.050% w/v brimonidine and either 0.025% to 0.035% w/v ketotifen fumarate (invention) or 0.3% w/v pheniramine maleate (reference) for 7 consecutive days.

### Result

The tested inventive formulations prophetically achieved greater reduction in cytokines and inflammation than ketotifen or pheniramine alone. Further, the tested inventive formulations prophetically reduced eye redness associated with ketotifen and pheniramine. Finally, the tested inventive formulations prophetically whitened eyes of the subject beyond the baseline whiteness.

## Claims

1. An ophthalmological composition for the treatment of allergies comprising brimonidine at a concentration from 0.025% to 0.035% w/v and ketotifen at a concentration from 0.025% to 0.7% w/v, wherein w/v denotes weight by total volume of the composition.

2. The composition of claim 1, further comprising a nonionic surfactant, hydroxypropylmethyl cellulose, a buffer and optionally, sorbate.

3. The ophthalmological composition of claim 2, wherein the nonionic surfactant is a polysorbate at a concentration from 1% w/v to 5% w/v.

4. The composition of claim 1 for use in a method of treating or preventing an allergic response comprising administering the composition of claim 1 to a subject in need thereof.

5. The composition of claim 1, wherein brimonidine is at a concentration of about 0.025% w/v.

6. The composition of claim 1, wherein the ophthalmological composition is formulated as an ophthalmic drop.

## Patentansprüche

1. Ophthalmologische Zusammensetzung für die Behandlung von Allergien, umfassend Brimonidin in einer Konzentration von 0,025 % bis 0,035 % w/v und Ketotifen in einer Konzentration von 0,025 % bis 0,7 % w/v, wobei w/v Gewicht nach Gesamtvolumen der Zusammensetzung bezeichnet.

2. Zusammensetzung nach Anspruch 1, ferner umfassend ein nichtionisches Tensid, Hydroxypropylmethylcellulose, einen Puffer und optional Sorbat.

3. Ophthalmologische Zusammensetzung nach Anspruch 2, wobei das nichtionische Tensid ein Polysorbat in einer Konzentration von 1 % w/v bis 5 % w/v ist.

4. Zusammensetzung nach Anspruch 1 zur Verwendung in einem Verfahren zum Behandeln oder Verhindern einer allergischen Reaktion, umfassend Verabreichen der Zusammensetzung nach Anspruch 1 an ein Subjekt, das dessen bedarf.

5. Die Zusammensetzung nach Anspruch 1, wobei Brimonidin in einer Konzentration von etwa 0,025 % w/v ist.

6. Zusammensetzung nach Anspruch 1, wobei die ophthalmologische Zusammensetzung als ein ophthalmologischer Tropfen formuliert ist.

## Revendications

1. Composition ophtalmologique pour le traitement des allergies comprenant de la brimonidine à une concentration de 0,025 % à 0,035 % p/v et du kétotifène à une concentration de 0,025 % à 0,7 % p/v, où p/v désigne le poids par volume total de la composition.

2. Composition de la revendication 1, comprenant en outre un tensioactif non ionique, de l'hydroxypropylméthylcellulose, un tampon et éventuellement un sorbate.

3. Composition ophtalmologique de la revendication 2, dans laquelle le tensioactif non ionique est un polysorbate à une concentration de 1 % p/v à 5 % p/v.

4. Composition de la revendication 1, destinée à être utilisée dans un procédé de traitement ou de prévention d'une réponse allergique, comprenant l'administration de la composition de la revendication 1 à un sujet qui en a besoin.

5. Composition de la revendication 1, dans laquelle la brimonidine est à une concentration d'environ 0,025 % p/v.

6. Composition de la revendication 1, dans laquelle la composition ophtalmologique est formulée sous la forme d'une goutte ophtalmique.
